(19) Europäisches Patentamt
European Patent Office
Office européen des brevets

(11) Veröffentlichungsnummer : **0 024 023 B2**

(12) **NEUE EUROPÄISCHE PATENTSCHRIFT**

(45) Veröffentlichungstag der neuen Patentschrift :
**11.11.92 Patentblatt 92/46**

(51) Int. Cl.$^5$ : **A61K 9/02,** A61K 31/415

(21) Anmeldenummer : **80104555.0**

(22) Anmeldetag : **01.08.80**

# (54) Antimykotische Mittel mit höherer Freisetzung der Wirkstoffe.

Die Akte enthält technische Angaben, die nach dem Eingang der Anmeldung eingereicht wurden und die nicht in dieserPatentschrift enthalten sind.

(30) Priorität : **11.08.79 DE 2932691**
**27.08.79 DE 2934542**

(43) Veröffentlichungstag der Anmeldung :
**18.02.81 Patentblatt 81/07**

(45) Bekanntmachung des Hinweises auf die Patenterteilung :
**19.06.85 Patentblatt 85/25**

(45) Bekanntmachung des Hinweises auf die Entscheidung über den Einspruch :
**11.11.92 Patentblatt 92/46**

(84) Benannte Vertragsstaaten :
**BE CH DE FR GB IT LI LU NL SE**

(56) Entgegenhaltungen :
**BE-A- 636 489**
**DE-A- 2 309 575**
**FR-A- 2 275 194**
**FR-A- 2 295 747**
**FR-A- 2 390 162**
**FR-M- 6 767**

(56) Entgegenhaltungen :
**GB-A- 860 877**
**US-A- 4 032 645**
**DAZ, 104, S. 950-955**
**J. Pharm.Pharmocol. 15 (T), Suppl. S 43T-55T (1963)**
**DAZ, 110 S. 942-946 (1970)**
**Vaginalmykosen. Therapie von heute im Trend von morgen. Canesten 1. Bayer AG 1987**

(73) Patentinhaber : **BAYER AG**
**W-5090 Leverkusen 1 Bayerwerk (DE)**

(72) Erfinder : **Plempel, Manfred, Dr.**
**Pahlkestrasse 5**
**W-5600 Wuppertal 1 (DE)**
Erfinder : **Bücheler, Manfred**
**Lorkenhöhe 49**
**W-5063 Overath (DE)**
Erfinder : **Gau, Wolfgang, Dr.**
**Am Eckbusch 39/56**
**W-5600 Wuppertal 1 (DE)**
Erfinder : **Regel, Erik**
**Bergerheide 72a**
**W-5600 Wuppertal 1 (DE)**
Erfinder : **Büchel, Karl Heinz, Prof. Dr.**
**Dabringhausener Strasse 42**
**W-5093 Burscheid (DE)**
Erfinder : **Ploschke, Hans-Jürgen**
**Neuenbaumerweg 4**
**W-5600 Wuppertal 1 (DE)**



Jouve, 18, rue Saint-Denis, 75001 PARIS

**Beschreibung**

Die Erfindung betrifft neuartige Formulierungen des bekannten antimykotischen Azolderivates Clotrimazol, die eine höhere Bioverfügbarkeit des Wirkstoffes aufweisen und dadurch eine Kurzzeittherapie ermöglichen.

Für die Behandlung von Vaginal-Infektionen mit Pilzen sind bereits Zubereitungen von antimykotischen Azolderivaten bekannt geworden, die Vaginaltabletten darstellen. Mit diesen Zubereitungen werden für eine vollständige Vaginal-Sanierung 14 bis 3 Tage Therapiezeit benötigt. Dies ist u.a. darauf zurückzuführen, daß in den üblichen Vaginaltablettenzubereitungen die enthaltenen Wirkstoffe nur zum Teil in wäßrigen Medien löslich sind.

Um zu einer Verkürzung der Therapiedauer zu kommen, benötigt man, besonders zur Eliminierung der Keime im Vaginalsekret, eine höhere Bioverfügbarkeit des Wirkstoffes im wäßrigen Milieu. Dafür sind die bekannten Formulierungen nur begrenzt geeignet, weil sich von dem vorhandenen Wirkstoffangebot nur ein kleiner Anteil im Flüssigkeitsvolumen der Vagina löst. Wenn man nun durch weitere Erhöhung der Wirkstoffkonzentration eine Verkürzung der Therapiedauer, z.B. auf einen Tag bei einmaliger Applikation, erreichen will, muß man für eine optimale Freisetzung des Wirkstoffes Sorge tragen.

Es wurde gefunden, daß Vaginaltablettenformulierungen, enthaltend Clotrimazol als Wirkstoff in Partikeln kleiner als 4 µm sowie übliche Tablettenhilfsstoffe und eine Puffersystem, das aus einer organischen Säure und einem ihrer Salze besteht, welche dadurch gekennzeichnet sind, daß das Puffersystem einen pH-Wert von 3 bis 4 und eine Ionenstärke von 0,1 bis 0,8 entfaltet, eine höhere Freisetzung des Wirkstoffes und damit eine verkürzte Therapiedauer von einem Tag durch das Erreichen von fungiziden Konzentrationen des Wirkstoffes ermöglichen.

Durch die Verminderung des mit den bekannten Formulierungen erhaltenen pH-Wertes von über 5 bis 6 auf 3 bis 4 in wäßriger Lösung kann eine Erhöhung des Wirkstofffreisetzung bis zu einer Zehnerpotenz erreicht werden.

Eine weitere Steigerung der Löslichkeit von Clotrimazol ergibt sich vermöge der Ionenstärke zwischen 0,1 bis 0,8 durch Ionenbindung.

Die Verträglichkeit solcher Formulierungen ist einwandfrei.

Puffersysteme beeinflussen die Zubereitungen in der angegebenen günstigen Weise. Solche können sein:

Citronensäure/Na-citrat primär
Milchsäure/Na-lactat
Weinsäure DL/K-Na-tartrat

Unter üblichen Tablettenhilfsstoffen werden hier die nachstehenden verstanden:
Stärke, z.B. Maisstärke, Reisstärke, Kartoffelstärke, Weizenstärke, Milchzucker, Glucose, Saccharose, mikrokristalline Cellulose, Siliciumdioxid kolloidal, Magnesiumstearat, Stearinsäure, Talcum, Polyvinylpyrrolidon (linear und quervernetzt), Natriumchlorid, Polyethylenglykol, Hydroxypropylmethylcellulose, Gelatine, Ca-Phosphate, Cellulose, Mannit, Natriumcarboxymethylstärke, Natriumcarbonat, Natriumbicarbonat, Calciumcarbonat, Natriumcarboxymethylcellulose (linear und quervernetzt), Magnesiumcarbonat.

Weitere Tablettenhilfsstoffe siehe "Die Tablette, Grundlagen und Praxis des Tablettierens, Granulierens und Dragierens" von W. A. Ritschel, S.85-144, sowie "Katalog pharmazeutischer Hilfsstoffe" verfaßt von einer Arbeitsgruppe der Firmen Ciba-Geigy, Hoffmann-La Roche und Sandoz, Basel 1974.

Aus GB-A-860877 ist es bekannt, durch Zusatz von Hilfsstoffen eine rasche Zerfallbarkeit von Zubereitungen und die Aufrechterhaltung eines physiologischen pH-Wertes im Vaginalmedium zu gewährleisten. Ferner ist es aus DE-A 2 309 575 bekannt, Säuren dafür zu verwenden, den physiologischen Säureschutz der Vagina aufrechtzuerhalten und die Anwendungsform "gewebsfreundlich" zu machen.

Aus FR-A-2 390 162 und US-A-4 032 645 sind Pufferzusätze zu Azolderivaten bekannt, die eine gute und schnelle Löslichkeit der Wirkstoffe gewährleisten. Es handelt sich dabei jedoch um Injektionslösungen, bei denen der Wirkstoff in einem geringen Volumen voll gelöst sein muß. Dagegen handelt es sich bei den erfindungsgemäßen Formulierungen um einen Wirkstoff, der in fester Form vorliegt und mittels dessen am Ort der Infektion durch Zerfall der Formulierung eine weitere Erhöhung der Wirkstoffkonzentration erreicht wird. Dieser Effekt ist im Hinblick auf die beiden Zitate überraschend. In DAZ, 104 (1964), 950-955, J. Pharm. Pharmacol. 15 (T), (1963), 43T-55T und DAZ 110 (1970), 942-946, werden Möglichkeiten der Freisetzung von Wirkstoffen zwecks besserer biopharmazeutischer Verfügbarkeit diskutiert. Insbesondere wird auch die Mikronisierung der Arzneistoffe erwähnt.

Die in dieser Literatur beschriebene Mikronisierung von Wirkstoffen zur Verbesserung der Bioverfügbarkeit beruht auf der Vergrößerung der Lösungsgeschwindigkeit.

Beispiel

500 g Clotrimazol werden in einem Wirbelschichtgranulator mit folgenden Mengen an Tablettenhilfsstoffen zunächst trocken gemischt: 190 g Citronensäure, 195 g Na-citrat prim., 314 g Milchzucker, 148 g Maisstärke, 230 g mikrokristalline Cellulose, 4 g Polyethylenglykol-Sorbitanoleat, dann wird durch Einsprühen von Wasser granuliert und getrocknet. Dann werden dem Granulat 34 g Mg-stearat und 85 g quervernetztes Polyvinylpyrrolidon zugemischt und zu

Vaginaltabletten mit einem Gesamtgewicht von 1700 mg tablettiert.

## Patentansprüche

1. Antimykotische Vaginaltabletten mit hoher Bioverfügbarkeit des Wirkstoffs, zur einmaligen Applikation enthaltend Clotrimazol der Formel

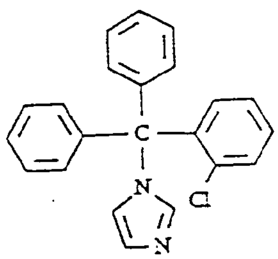

als Wirkstoff in Partikeln kleiner als 4 μm sowie übliche Tablettenhilfsstoffe und ein Puffersystem der aus einer organischen Säure und einem ihrer Salze besteht, dadurch gekennzeichnet, daß das Puffersystem einen pH-Wert von 3 bis 4 und eine Ionenstärke von 0,1 bis 0,8 entfaltet.

2. Antimykotische Vaginaltabletten nach Anspruch 1, dadurch gekennzeichnet, daß das Puffersystem aus Citronensäure und primärem Natriumcitrat besteht.

3. Antimykotische Vaginaltabletten nach Anspruch 1, dadurch gekennzeichnet, daß das Puffersystem aus Milchsäure und Calciumlactat besteht.

## Claims

1. Antimycotic vaginal tablets with a high bioavailability of the active compound, for a single administration, containing clotrimazole of the formula

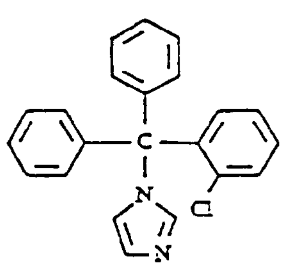

as the active compound in particles smaller than 4 μm as well as customary tablet auxiliaries and a buffer system consisting of an organic acid and of one of the salts thereof, characterised in that the buffer system produces a pH of 3 to 4 and an ionic strength of 0.1 to 0.8.

2. Antimycotic vaginal tablets according to Claim 1, characterised in that the buffer system consists of citric acid and primary sodium citrate.

3. Antimycotic vaginal tablets according to Claim 1, characterised in that the buffer system consists of lactic acid and calcium lactate.

## Revendications

1. Comprimés vaginaux antimycotiques avec une grande biodisponibilité de la substance active, pour une administration unique, contenant du clotrimazol de formule

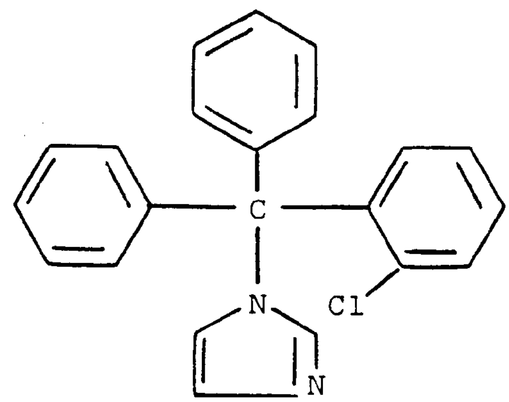

comme substance active en particules inférieures à 4 μm ainsi que des substances auxiliaires habituelles pour comprimés et un système tampon constitué d'un acide organique et d'un de ses sels, caractérisés en ce que le système tampon produit un pH de 3 à 4 et une intensité ionique de 0,1 à 0,8.

2. Comprimés vaginaux antimycotiques selon la revendication 1, caractérisés en ce que le système tampon est constitué d'acide citrique et de citrate de sodium primaire.

3. Comprimés vaginaux antimycotiques selon la revendication 1, caractérisés en ce que le système tampon est constitué d'acide lactique et de lactate de calcium.